**Europäisches Patentamt**

(19) **European Patent Office**

**Office européen des brevets**

(11) Veröffentlichungsnummer: **0 041 107**
**B1**

(12) # EUROPÄISCHE PATENTSCHRIFT

(45) Veröffentlichungstag der Patentschrift:
**21.12.83**

(51) Int. Cl.³: **A 61 B 3/12**

(21) Anmeldenummer: **81102040.3**

(22) Anmeldetag: **19.03.81**

(54) **Binokulares Ophthalmoskop.**

(30) Priorität: **31.05.80 DE 3020750**

(43) Veröffentlichungstag der Anmeldung:
**09.12.81 Patentblatt 81/49**

(45) Bekanntmachung des Hinweises auf die Patenterteilung:
**21.12.83 Patentblatt 83/51**

(84) Benannte Vertragsstaaten:
**FR GB IT**

(56) Entgegenhaltungen:
**DE - A - 2 810 539**
**GB - A - 1 473 537**
**US - A - 3 405 994**
**US - A - 3 582 191**
**US - A - 3 944 343**
**US - A - 3 963 329**

(73) Patentinhaber: **Kocher, Jakob, Wiesenweg 19,
D-7401 Dusslingen (DE)**

(72) Erfinder: **Foerster, Michael, Dr. med., In der Borbeck 23,
D-4300 Essen 16 (DE)**
Erfinder: **Kocher, Jakob, Wiesenweg 19,
D-7401 Dusslingen (DE)**

(74) Vertreter: **Grams, Klaus Dieter, Dipl.-Ing. et al,
Patentanwaltsbüro Tiedtke-Bühling-Kinne-
Grupe-Pellmann-Grams Bavariaring 4,
D-8000 München 2 (DE)**

## Binokulares Ophthalmoskop

Die Erfindung bezieht sich auf ein binokulares Ophthalmoskop gemäß dem Oberbegriff von Patentanspruch 1.

Ein solches Ophthalmoskop ist bekannt (DE-OS 2 534 784). Es dient zum beidäugigen Beobachten des Hintergrundes eines zu untersuchenden Auges, wobei außer dem Ophthalmoskop, das der Beobachter vor seinen Augen trägt, eine Ophthalmoskoplupe zur Anwendung kommt, die vor das zu untersuchende Auge gehalten wird, das sich in einem Abstand von ca. 50 cm vom Ophthalmoskop befindet.

Auf das zu untersuchende Auge sind der Beleuchtungsstrahlengang und die den beiden Augen des Beobachters zugeordneten beiden Beobachtungsstrahlengänge gerichtet. Der Beleuchtungsstrahlengang und die beiden Beobachtungsstrahlengänge müssen voneinander getrennt sein, damit die Ophthalmoskopie möglichst reflexfrei durchgeführt werden kann. Damit trotz der Enge der Pupille des zu beobachtenden Auges überhaupt eine beidäugige Beobachtung des Auges im Abstand von ca. 50 cm bzw. des von der Ophthalmoskopielupe entworfenen virtuellen Bildes möglich ist, muß der Konvergenzwinkel der beiden Beobachtungsstrahlengänge, d. h. der von den auf einen Punkt des virtuellen Bildes fixierten Beobachtungsstrahlengängen eingeschlossene Winkel mittels der Beobachtungseinheit verringert werden, wobei allerdings ein unter Berücksichtigung dieser Forderung möglichst großer Konvergenzwinkel zweckmäßig ist, um ein möglichst gut stereokopisches Bild des Augenhintergrundes zu erhalten.

Bei dem bekannten Ophthalmoskop sind die zweiten Spiegel und diesen zugeordnete Okulare in Querrichtung verschiebbar, damit einerseits der Okularabstand auf die Pupillardistanzweite des Beobachters einstellbar ist und damit andererseits der Konvergenzwinkel verringert werden kann, wozu die beiden zweiten Spiegel und die Okulare noch weiter auseinandergeschoben werden als es der Pupillardistanzweite des Beobachters entspricht. Diese Verringerung des Konvergenzwinkels soll es ermöglichen, auch bei einer sehr engen Pupille des zu beobachtenden Auges mit einem Durchmesser von beispielsweise 1 mm eine beidäugige Beobachtung des Augenhintergrundes durchzuführen. Nachteilig bei dem bekannten Ophthalmoskop ist jedoch die Gesichtsfeldeinschränkung des Beobachters. Ferner ist bei dem bekannten Ophthalmoskop vorgesehen, daß der dritte Spiegel um eine zur Beobachtungsebene parallele Achse schwenkbar ist, was es ermöglicht, die Richtung des Beleuchtungsstrahlenganges zu justieren. Die dadurch erreichbare Reflexminderung ist jedoch noch unzureichend.

Der Erfindung liegt die Aufgabe zugrunde, das gattungsgemäße Ophthalmoskop derart auszubilden, daß auch unter ungünstigen Bedingungen, wie beispielsweise geringer Pupillenweite und reflexfördernder Krümmung der Hornhaut des zu beobachtenden Auges, noch eine möglichst zuverlässige Ophthalmoskopie möglich ist.

Diese Aufgabe wird erfindungsgemäß dadurch gelöst, daß die zwei ersten Spiegel innerhalb der Beobachtungseinheit gemeinsam zum und vom zu beobachtenden Auge in der Beobachtungsebene verschiebbar sind und daß der Beleuchtungsstrahlengang in einer auf der Beobachtungsebene senkrecht stehenden Richtung zu der und von der Beobachtungsebene verschiebbar ist.

Beim erfindungsgemäßen Ophthalmoskop können die beiden zentral angeordneten ersten Spiegel gemeinsam verschoben werden, was eine Änderung des Konvergenzwinkels zur Folge hat und somit eine Einstellung des optimalen Konvergenzwinkels ermöglicht, ohne daß Gesichtsfeldeinschränkungen des Beobachters in Kauf genommen zu werden brauchen. Zudem ist die Bedienung vereinfacht, da zur Änderung des Konvergenzwinkels lediglich eine Einstellung vorgenommen zu werden braucht und nicht beide Okulare mit den zugeordneten Spiegeln unabhängig voneinander verschoben werden müssen. Dadurch, daß der Beleuchtungsstrahlengang zu der und von der Beobachtungsebene verschiebbar ist, kann der Beleuchtungsstrahlengang so nahe an die Beobachtungsebene gebracht werden, daß auch bei engen Pupillenweiten des zu beobachtenden Auges eine ausreichende Lichtmenge auf den beobachteten Bereich geworfen wird, und kann der Beleuchtungsstrahlengang — sofern die Pupillenweite dies zuläßt — von der Beobachtungsebene so weit entfernt werden, daß größtmögliche Reflexfreiheit erreicht wird.

Die Verschiebbarkeit des Beleuchtungsstrahlenganges zu der und von der Beobachtungsebene kann dadurch gewährleistet sein, daß der dritte Spiegel in der Beleuchtungseinheit im wesentlichen senkrecht zur Beobachtungsebene verschiebbar ist. Vorzugsweise wird jedoch die gesamte Beleuchtungseinheit relativ zur Beobachtungseinheit verschoben, wozu vorgesehen sein kann, daß die Verbindungseinrichtung einen an der Beobachtungseinheit befestigten, im wesentlichen senkrecht zur Beobachtungsebene verlaufenden Arm aufweist, der verschiebbar in einer Führung an der Beleuchtungseinheit sitzt. Dies bringt den Vorteil mit sich, daß die Verschiebung einfach durchführbar ist, da lediglich die gesamte Beleuchtungseinheit erfaßt und verschoben zu werden braucht.

In vorteilhafter Ausbildung der Erfindung kann ferner vorgesehen sein, daß der dritte Spiegel oder ein in der Beleuchtungseinheit angeordneter vierter Spiegel um eine zur Beobachtungsebene parallele Achse schwenkbar ist. Dies ermöglicht einerseits eine Einstellung der Richtung des Beleuchtungsstrahlenganges, so daß dieser nach seiner Verschiebung relativ zur Beobachtungsebene erneut ausgerichtet werden kann und dadurch der Winkel zwischen dem Beleuchtungsstrahlengang und der Beobachtungsebene verändert werden kann, und ermöglicht andererseits, den Beleuchtungsstrahlengang innerhalb eines gewissen Winkelbereiches zu neigen, so daß unterschiedliche Anteile des Beleuchtungsstrahlenbündels in die Pupille des untersuchten Auges geleitet werden können. Dies ermöglicht eine Reflexverringerung.

Vorzugsweise umfaßt das erfindungsgemäße Ophthalmoskop eine im Gehäuse der Beobachtungseinheit ausgebildete Führungsnut, die parallel zur Winkelhalbierenden der Beobachtungsstrahlengänge verläuft, einen Träger, an dem die zwei ersten Spiegel befestigt sind, einen am Träger ausgebildeten Vorsprung, der in die Nut eingreift, und ein außen an der Beobachtungseinheit angeordnetes Betätigungselement, das mit dem Träger fest verbunden ist. Die Nut und der Ansatz am Träger der beiden ersten Spiegel führen diese bei ihrer Verschiebung zuverlässig und verhindern ein Verkanten der beiden ersten Spiegel.

Schließlich können in vorteilhafter Ausbildung der Erfindung eine verstellbare Blende und eine Objektivlinse in der Beobachtungseinheit vorgesehen sein, wobei die Blende in der dingseitigen Brennebene der Objektivlinse angeordnet ist. Aufgrund dieser Ausbildung wird nicht die Wendel der Lichtquelle in die Pupille des zu beobachtenden Auges abgebildet sondern das Bild der verstellbaren Blende. Dies führt zu sehr hoher Reflexfreiheit des Bildes des Augenhintergrundes. Zudem kann durch Verkleinern der Apertur der verstellbaren Blende der Streulichtanteil so weit reduziert werden, daß auch bei teilweise durchgetrübter Linse des zu untersuchenden Auges noch eine Ophthalmoskopie möglich ist.

Weitere Vorteile und Merkmale der Erfindung ergeben sich aus den Unteransprüchen sowie der folgenden Beschreibung eines bevorzugten Ausführungsbeispieles unter Bezugnahme auf die Zeichnungen. Es zeigt

Figur 1 eine perspektivische Ansicht der bevorzugten Ausführungsform des erfindungsgemäßen binokularen Ophthalmoskops;

Figur 2 in zeichnerisch vereinfacher Darstellung einen Schnitt gemäß A-A in Figur 3 durch das Ophthalmoskop;

Figur 3 in zeichnerisch vereinfachter Darstellung einen Schnitt gemäß B-B in Figur 2; und

Figur 4 eine Ansicht von Elementen einer Verbindungseinrichtung des Ophthalmoskops.

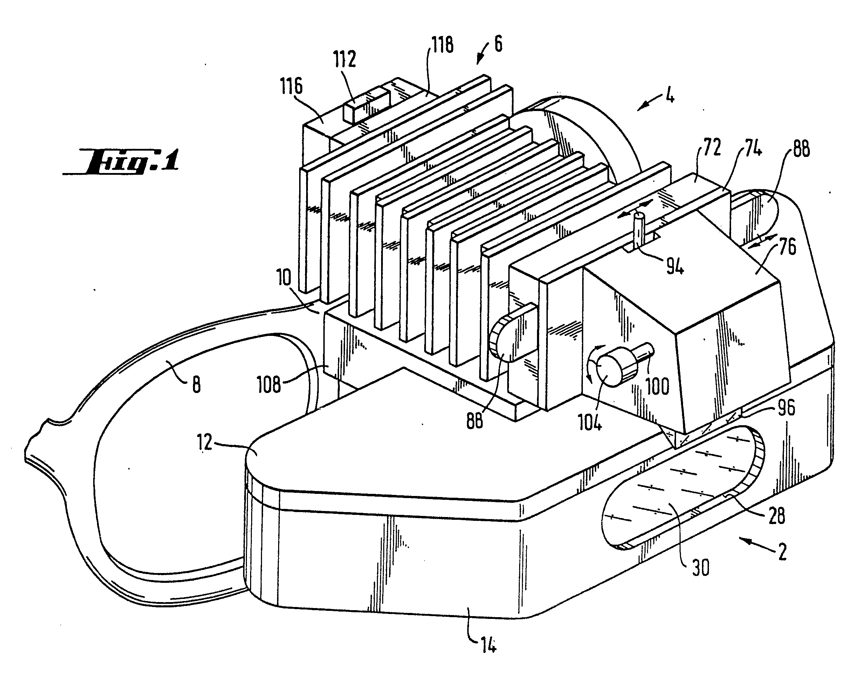

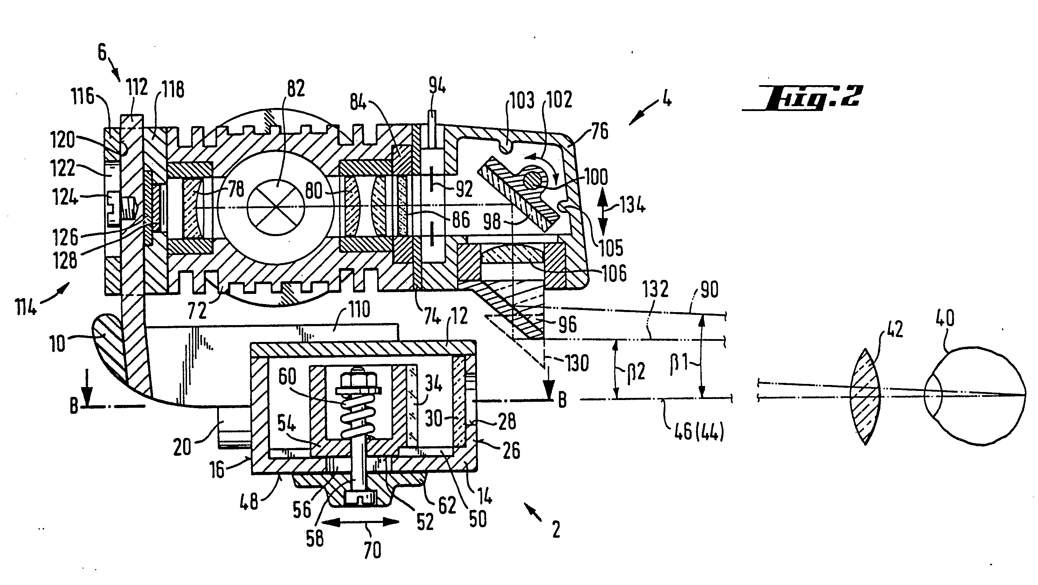

Das dargestellte Ophthalmoskop umfaßt eine Beobachtungseinheit 2 und eine Beleuchtungseinheit 4, die durch eine Verbindungseinrichtung 6 miteinander verbunden sind. Die Verbindungseinrichtung 6 stellt außerdem die Verbindung zwischen der Beobachtungseinheit 2 und der Beleuchtungseinheit 4 einerseits und einem Brillengestell 8 andererseits her, mittels dessen das Ophthalmoskop vom Beobachter wie eine Brille getragen werden kann. Die Verbindungseinrichtung 6 ist mit dem Brillengestell 8 im Bereich von dessen Nasensteg 10 auf nicht dargestellte Weise fest verbunden (siehe Figur 2).

Das Brillengestell 8 kann für den Fall, daß der Beobachter fehlsichtig ist, übliche Korrektionsgläser tragen. Obwohl das erfindungsgemäße Ophthalmoskop vorzugsweise in dargestellter Weise als sogenanntes Brillenophthalmoskop ausgebildet ist, können die Beobachtungseinheit 2, die Beleuchtungseinheit 4 und die Verbindungseinrichtung 6 auch mit Hilfe anderer Mittel, beispielsweise eines üblichen Stirnreifes, am Kopf des Beobachters gehalten werden.

Die Beobachtungseinheit 2 umfaßt ein mit Hilfe eines Deckels 12 verschlossenes Gehäuse 14, in dessen Rückwand 16 zwei Okulare 18 und 20 eingesetzt sind, deren optische Achsen einen der Pupillardistanzweite des Beobachters angepaßten Abstand haben. Obwohl es möglich ist, die Okulare 18 und 20 am Gehäuse 14 derart verschiebbar anzuordnen, daß sie auf die Pupillardistanzweiten des Beobachters einstellbar sind, wird es unter praktischen Gesichtspunkten der Benutzung für zweckmäßiger gehalten, die Okulare 18 und 20 am Gehäuse 14 fest anzuordnen und mehrere, beispielsweise vier Beobachtungseinheiten mit unterschiedlichen Pupillardistanzweiten vorzusehen. Um dem Beobachter akkomodationsloses Sehen in einem Abstand von ca. 50 cm zu ermöglichen, sind in die Okulare 18 und 20 Okularlinsen 22 und 24 eingesetzt.

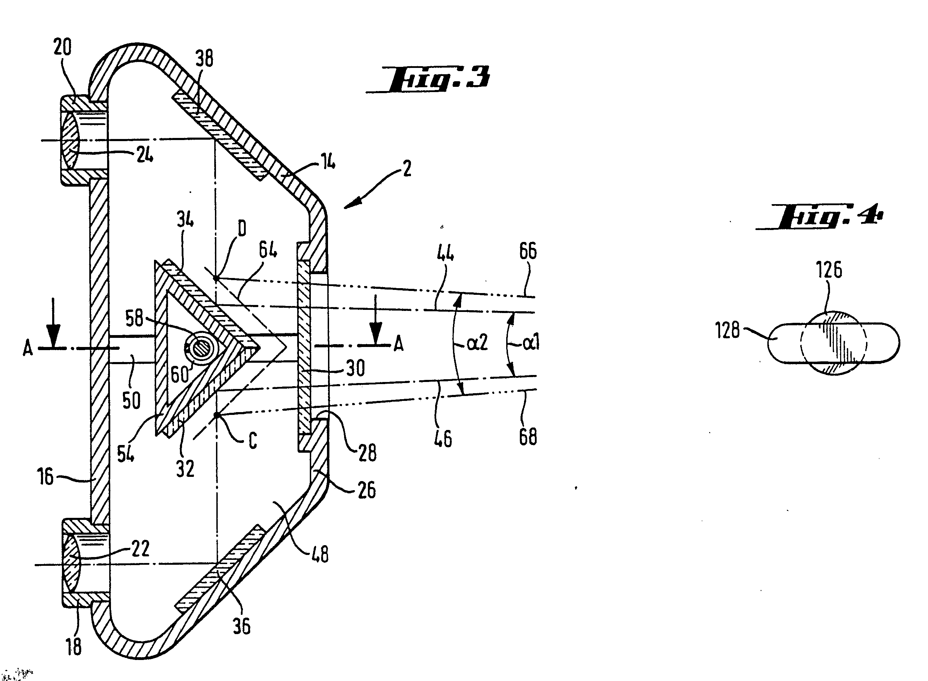

In der zur Rückwand 16 parallelen Vorderwand 26 des Gehäuses 14 ist eine längliche Öffnung 28 ausgebildet, die durch ein durchsichtiges Planglas 30 verschlossen ist. Innerhalb des Gehäuses 14 sind hinter dem Planglas 30 zwei erste Spiegel 32 und 34 derart angeordnet, daß ihre spiegelnden Oberflächen einen Keil mit einem Keilwinkel von 90° bilden, daß die Keilspitze dem Planglas 30 zugewandt ist, daß die ersten Spiegel 32 und 34 senkrecht zu der durch die optischen Achsen der Okulare 18 und 20 definierten Ebene verlaufen und daß sie symmetrisch zu der mit der Schnittebene A-A zusammenfallenden senkrechten Mittelebene der Beobachtungseinheit 2 angeordnet sind. Parallel zu jedem der ersten Spiegel 32 und 34 ist auf jeder der optischen Achsen der beiden Okulare 18 und 20 jeweils ein zweiter Spiegel 36 bzw. 38 im Gehäuse 14 unverschiebbar befestigt. Während der Beobachtung eines in Figur 2 schematisch dargestellten zu untersuchenden Auges 40 mittels des Ophthalmoskops sowie einer Ophthalmoskoplupe 42 sind die beiden Augen des Beobachters auf das zu beobachtende Auge bzw. — genauer — auf das von der Ophthalmoskoplupe 42 entworfene virtuelle Bild gerichtet. Bei der in Figur 3 dargestellten Stellung der beiden ersten Spiegel 32 und 34 haben die den beiden Augen des Beobachters zugeordneten Beobachtungsstrahlengänge, die mit 44 bzw. 46 bezeichnet sind, den durch die strichpunktierten Linien gezeigten Verlauf. Die beiden Beobachtungsstrahlengänge 44 und 46 schließen einen Konvergenzwinkel $\alpha 1$ ein. Jeder der Beobachtungsstrahlengänge 44 und 46 verläuft durch die Planscheibe 30 und wird vom ersten Spiegel 32 bzw. 34 und vom zweiten Spiegel 36 bzw. 38 so umgelenkt, daß der Abstand der Beobachtungsstrahlengänge beobachterseitig gleich der Pupillardistanzweite des Beobachters bzw. dem Abstand der optischen Achsen der Okulare 18 und 20 ist. Die beiden Beobachtungsstrahlengänge 44 und 46 definieren die Beobachtungsebene, die für Figur 3 mit der Zeichenebene zusammenfällt.

Um den Konvergenzwinkel zwischen den Beobachtungsstrahlengängen ändern zu können, insbesondere enger Pupillenweite des zu beobachtenden Auges anpassen zu können, andererseits jedoch so groß wie möglich zu machen, um optimale Stereoskopie zu erreichen, sind die beiden ersten Spiegel 32 und 34 innerhalb der Beobachtungseinheit 2 gemeinsam in der Beobachtungsebene verschiebbar. Zu diesem Zweck ist im Boden 48 des Gehäuses 14 eine Führungsnut 50 ausgebildet, in der ein Vorsprung 52 verschiebbar sitzt, der am Boden eines Trägers 54 ausgebildet ist, an dem die beiden ersten Spiegel 32 und 34 befestigt sind. Der Träger 54 hat das in Figur 3 erkennbar dreieckige Profil in Draufsicht. Die Nut 50 verläuft parallel zur Beobachtungsebene in Richtung der Winkelhalbierenden der beiden Beobachtungsstrahlengänge 44 und 46, d. h. senkrecht zur Rückwand 16 der Beobachtungseinheit 2. Im Boden 48 ist unterhalb der Nut 50 und in gleicher Richtung wie diese verlaufend ein Langschlitz 56 ausgebildet (siehe Figur 2). Durch den Langschlitz 56 und eine nicht bezeichnete Bohrung im Boden des Trägers 54 verläuft eine Schraube 58, auf die eine Mutter geschraubt ist, an der sich über einen Federsitz eine Feder 60 abstützt, deren anderes Ende sich am Boden des Trägers 54 abstützt. Der Kopf der Schraube 58 sitzt in einer Ausnehmung in einem als Kreisscheibe ausgebildeten Betätigungselement 62, das auf der Außenseite des Bodens 48 des Gehäuses 14 angeordnet ist. Aufgrund der vorstehend beschriebenen Ausbildung wird der Träger 54 gegen die Innenseite des Bodens 48 und das Betätigungselement 62 gegen die Außenseite des Bodens 48 elastisch gedrückt, wobei es möglich ist, mittels des Betätigungselementes 62 den Träger 54 und die an ihm befestigten ersten Spiegel 32 und 34 entlang der Führungsnut 50 zu verschieben. Die Verschiebung nach vorne und nach hinten, d. h. nach links bzw. rechts in den Figuren 2 und 3, ist durch Anschlag der Schraube 58 an den Enden des Langschlitzes 56 begrenzt. Das Betätigungselement 62 hat einen solchen Durchmesser, daß es den Langschlitz 56 auch dann noch bedeckt, wenn sich der Träger 54 in einer seiner beiden Anschlagstellungen befindet.

Wenn der Träger 54 und mit ihm die beiden ersten Spiegel 32 und 34 aus der in den Figuren 2 und 3 dargestellten Stellung so nach vorne verschoben ist, daß die Spiegeloberflächen die in Figur 3 gestrichelt dargestellte Stellung 64 einnehmen, haben die für diesen Fall mit 66 und 68 bezeichneten Beobachtungsstrahlengänge zwischen den ersten Spiegeln und dem beobachteten Auge den durch Linien mit vier Punkten angedeuteten Verlauf. Wegen der dabei vergrößerten Basis (Abstand zwischen den Punkten C und D) bei gleichem Beobachtungsabstand schließen die Beobachtungsstrahlengänge 66 und 68 einen Konvergenzwinkel $\alpha 2$ ein, der größer als der Konvergenzwinkel $\alpha 1$ ist. Wenn der Träger 54 mit den beiden ersten Spiegeln 32 und 34 aus der in Figur 3 dargestellten Stellung nach links verschoben wird, verkleinert sich der Konvergenzwinkel auf entsprechende Weise. Es ist somit erkennbar, daß durch die genannte Verschiebung der beiden ersten Spiegel 32 und 34 eine Änderung des Konvergenzwinkels zwischen den Beobachtungsstrahlengängen und somit jeweils eine optimale Anpassung des Konvergenzwinkels an den Beobachtungsfall möglich ist. Hierzu ist es lediglich erforderlich, das Betätigungselement 62 in Richtung des in Figur 2 eingezeichneten Doppelpfeiles 70 vor- oder zurückzuschieben.

Die Öffnung 28 in der Vorderwand 26 des Gehäuses 14 hat eine solche Länge, daß auch bei dem größten einstellbaren Konvergenzwinkel die Sicht durch die Öffnung 28 unbehindert ist.

Praktisch kann die vorstehend beschriebene Beobachtungseinheit 2 derart dimensioniert sein, daß der Konvergenzwinkel stufenlos zwischen 0,86° und 2,64° veränderbar ist.

Mittig oberhalb der Beobachtungseinheit 2 ist die Beleuchtungseinheit 4 angeordnet. Diese umfaßt ein mit Kühlrippen versehenes Gehäuseteil 72, an das vorne, d. h. rechts in Figur 2, über eine Zwischenplatte 74 ein weiteres Gehäuseteil 76 angesetzt ist. Im Gehäuseteil 72 ist zwischen einem Reflektor bzw. Hohlspiegel 78, der am in Figur 2 linken Ende der Beleuchtungseinheit 4 angeordnet ist, und einem Kondensor 80 eine beispielsweise als Halogenlampe ausgebildete Lichtquelle 82 angeordnet. Der elektrische Anschluß der Lichtquelle 82 ist in den Figuren nicht dargestellt. Die optische Achse des Hohlspiegels 78 und des Kondensors 80 verläuft im wesentlichen parallel zur Beobachtungsebene und somit während der Benutzung des Ophthalmoskops im wesentlichen waagerecht, so daß die gesamte Beleuchtungseinheit 4 ihre Haupterstreckung in horizontaler Richtung hat, was zu günstiger Schwerpunktlage des Ophthalmoskops nahe dem tragenden Brillengestell 8 führt.

Zwischen dem Gehäuseteil 72 und der Zwischenplatte 74 ist verschiebbar ein Schieber 84 geführt, der ein Farbfilter 86 oder mehrere Farbfilter trägt, die mittels der aus der Beleuchtungseinheit 4 vorstehenden Enden 88 des Schiebers 84 wahlweise in den Beleuchtungsstrahlengang gebracht werden können. Der Beleuchtungsstrahlengang ist in Figur 2 mit 90 bezeichnet und durch eine seinen Zentralstrahl wiedergebende, mit zwei Punkten unterbrochene Linie dargestllt. Vor dem Farbfilter 86, d. h. rechts von diesem in Figur 2, ist eine als Irisblende ausgebildete verstellbare Blende 92 angeordnet, deren Apertur mittels eines aus der Beleuchtungseinheit 4 vorstehenden Blendenstellhebels 94 einstellbar ist.

Am vorderen Ende der Beleuchtungseinheit 4 ist an dessen Unterseite ein als Umkehrprisma ausgebildeter dritter Spiegel 96 angeordnet, dessen spiegelnde Flächen einen Winkel von ungefähr 45° mit der durch die Beobachtungsstrahlengänge definierten Beobachtungsebene einschließt. Zu diesem dritten Spiegel 96 wird der Beleuchtungsstrahlengang von einem im Gehäuseteil 76 angeordneten vierten Spiegel 98 umgelenkt. Der vierte Spiegel 98 ist mittels einer drehbar im Gehäuseteil 76 gelagerten Achse 100 schwenkbar, wie dies durch einen Doppelpfeil 102 in Figur 2 angedeutet ist. Der dritte Spiegel 96 und der vierte Spiegel 98 sind derart ausgerichtet, daß der unten aus der Beleuchtungseinheit 4 austretende Beleuchtungsstrahlengang 90 auf das zu beobachtende Auge 40 gerichtet ist und in einer zur Beobachtungsebene senkrechten, die Winkelhalbierende zwischen den beiden Beobachtungsstrahlengängen 44 und 46 einschließenden Ebene verläuft.

Die Achse 100 ragt aus dem Gehäuseteil 76 heraus und trägt an ihrem freien Ende einen Betätigungsknopf 104, mittels dessen die Schwenkstellung des vierten Spiegels 98 und somit die Richtung des Beleuchtungsstrahlenganges 90 eingestellt werden kann. Dabei ermöglicht es die Schwenkbarkeit des vierten Spiegels 98 nicht nur, den Beleuchtungsstrahlengang 90 auf das zu beobachtende Auge zu richten, sondern sie ermöglicht es auch, die Richtung des Beleuchtungsstrahlenganges 90 in einem Winkelbereich von ca. 10° so zu verändern, daß das zu beobachtende Auge von unterschiedlichen Anteilen des Beleuchtungsstrahlenbündels getroffen wird, was eine Anpassung an die Reflexionsbedingungen des zu beobachtenden Auges ermöglicht. Der Schwenkbereich des vierten Spiegels 98 ist durch Anschläge 103 und 105 am Gehäuseteil 76 begrenzt.

Zwischen dem dritten Spiegel 96 und dem vierten Spiegel 98 ist im Gehäuseteil 76 im Beleuchtungsstrahlengang eine Objektivlinse 106 befestigt, die der Blende 92 so zugeordnet ist, daß sich die Blende 92 ungefähr in der dingseitigen Brennebene der Objektivlinse 106 befindet. Dies führt dazu, daß in der Pupille des zu beobachtenden Auges nicht die Lichtquelle 82 sondern die Blende 92 abgebildet wird, was erheblich zur Reflexfreiheit des Bildes des Augenhintergrundes beiträgt. Durch Verkleinern der verstellbaren Blende 92 kann der Streulichtanteil im Beleuchtungsstrahlenbündel so weit verringert werden, daß auch bei teilweise durchgetrübter Linse des zu beobachtenden Auges noch eine Ophthalmoskopie möglich ist.

Die Verbindungseinrichtung 6 zwischen der Beobachtungseinheit 2 und der Beleuchtungseinheit 4 umfaßt zwei zueinander parallele, im wesentlichen horizontal verlaufende Arme 108 und 110, an deren vorderen Enden die Beobachtungseinheit 2 befestigt ist. Die beiden Arme 108 und 110 sind fest verbunden oder einstückig ausgebildet mit einem weiteren Arm 112, der im wesentlichen senkrecht zur Beobachtungsebene verläuft und in seinem in Figur 2 oberen Abschnitt einen gleichbleibenden Rechteckquerschnitt hat. Die drei Arme 108, 110 und 112 sind mit dem Nasensteg 10 des Brillengestelles 8 beispielsweise mit Hilfe von nicht dargestellten Schrauben fest verbunden. Zur Verbindungseinrichtung 6 gehört ferner eine Führung 114, die am hinteren, d. h. in Figur 2 linken Ende der Beleuchtungseinheit 4 befestigt ist und den Arm 112 verschiebbar aufnimmt. In der Führung 114 ist zwischen zwei Platten 116 und 118 eine Nut 120 ausgebildet, in die der Arm 112 eingeschoben ist. In der Platte 116 ist ein in Längsrichtung der Nut 120 verlaufender Langschlitz 122 ausgebildet, durch den hindurch eine Kopfschraube 124 in den Arm 112 geschraubt ist, die zusammen mit dem Langschlitz 122 die Relativverschiebung zwischen dem Arm 112 und der Führung 114 begrenzt. Im von der Platte 118 gebildeten Boden der Nut 120 sitzt eine kreisförmige Druckscheibe 126, die von einer als Blattfeder ausgebildeten Feder 128 gegen den Arm 112 gedrückt wird. Eine Ansicht von Druckscheibe 126 und Feder 128 zeigt Figur 4. Die von der Feder 128 elastisch gegen den Arm 112 gedrückte Druckscheibe 126 sorgt für einen der Verschiebung des Armes 112 in der Führung 114 entgegenwirkenden gewissen Widerstand.

Aufgrund der vorstehend beschriebenen Ausbildung der Verbindungseinrichtung 6 kann die gesamte Beleuchtungseinheit 4 relativ zur Beobachtungseinheit 2 und somit zur Beobachtungsebene senkrecht nach oben und unten verschoben werden. Diese Verschiebung bewirkt eine Verschiebung des Beleuchtungsstrahlenganges 90 zur Beobachtungsebene bzw. von dieser weg. In Figur 2 ist der dritte Spiegel 96 in einer Stellung 130 gestrichelt für den Fall dargestellt, daß die Beleuchtungseinheit 4 aus der in Figur 2 dargestellten Stellung nach unten verschoben ist. Es ist erkennbar, daß dabei der für diesen Fall mit 132 bezeichnete und durch eine den Zentralstrahl wiedergebende, mit drei Punkten unterbrochene Linie dargestellte Beleuchtungsstrahlengang näher an den durch die Beobachtungsstrahlengänge 44 und 46 definierten Beobachtungsebene verläuft als der Beleuchtungsstrahlengang 90. Durch Verstellen des vierten Spiegels 98 kann der Beleuchtungsstrahlengang 132 auf den gleichen Punkt gerichtet werden wie der Beleuchtungsstrahlengang 90, wobei dann der Winkel $\beta2$ zwischen dem Beleuchtungsstrahlengang 132 und der Beobachtungsebene kleiner ist als der Winkel $\beta1$ zwischen dem Beleuchtungsstrahlengang 90 und der Beobachtungsebene. Durch das Verschieben der gesamten Beleuchtungseinheit 4 in Richtung eines Doppelpfeiles 134 ist es somit möglich, den Abstand zwischen dem Beleuchtungsstrahlengang und der Beobachtungsebene und in Verbindung mit entsprechender Justierung des vierten Spiegels 98 den Winkel zwischen dem Beleuchtungsstrahlengang und der Beobachtungsebene so zu ändern, daß unter Berücksichtigung der Beobachtungsbedingungen eine maximal reflexfreie Ophthalmoskopie auch bei Augen mit enger Pupillenweite möglich ist.

Bei einem praktisch ausgeführten Ophthalmoskop kann der Winkel zwischen dem Beleuchtungsstrahlengang und der Beobachtungsebene beispielsweise in einem Bereich zwischen 1,5° und 2,5° verstellt werden.

Die Erfindung ist nicht auf das vorstehend beschriebene Ausführungsbeipiel beschränkt. Beispielsweise kann statt des vierten Spiegels 98 der dritte Spiegel 96 um eine zur Beobachtungsebene parallele Achse schwenkbar ausgebildet sein. Ferner ist es auch möglich, den dritten Spiegel 96 in Richtung des Doppelpfeiles 134 verschiebbar in der Beleuchtungseinheit anzuordnen, statt die gesamte Beleuchtungseinheit 4 relativ zur Beobachtungseinheit 2 verschiebbar auszubilden.

**Patentansprüche**

1. Binokulares Ophthalmoskop zur indirekten Beobachtung, mit einer Beobachtungseinheit (2) und einer Beleuchtungseinheit (4), die mit der Beobachtungseinheit mittels einer Verbindungseinrichtung (6) verbunden ist, wobei die Beobachtungseinheit (2) zwei dicht beieinander in die Beobachtungseinheit eintretende, eine Beobachtungsebene definierende Beobachtungsstrahlengänge (44, 46; 66, 68) jeweils mit Hilfe eines ersten (32, 34) und eines zweiten Spiegels (36, 38) derart umlenkt, daß ihr Abstand auf die Pupillardistanzweite des Beobachters vergrößert ist, wobei die beiden ersten Spiegel (32, 34) zwischen den beiden zweiten Spiegeln (36, 38) unter Bildung eines Keils dicht beieinander angeordnet sind und wobei die Beleuchtungseinheit (2) eine Lichtquelle (82) und einen dritten, austrittsseitigen Spiegel (96) umfaßt, der Licht von der Lichtquelle entlang einem Beleuchtungsstrahlengang (90, 132) zum zu beobachtenden Auge richtet, dadurch gekennzeichnet, daß die zwei ersten Spiegel (32, 34) innerhalb der Beobachtungseinheit (2) gemeinsam zum und vom zu beobachtenden Auge in der Beobachtungsebene verschiebbar sind und daß der Beleuchtungsstrahlengang (90, 132) in einer auf der Beobachtungsebene senkrecht stehenden Richtung zu der und von der Beobachtungsebene verschiebbar ist.

2. Ophthalmoskop nach Anspruch 1, dadurch gekennzeichnet, daß der dritte Spiegel (96) um eine zur Beobachtungsebene parallele Achse schwenkbar ist.

3. Ophthalmoskop nach Anspruch 1, dadurch gekennzeichnet, daß zwischen der Lichtquelle (82) und dem dritten Spiegel (96) ein vierter Spiegel (98) in der Beleuchtungseinheit (4) angeordnet ist, der um eine zur Beobachtungsebene parallele Achse (100) schwenkbar ist.

4. Ophthalmoskop nach einem der Ansprüche 1 bis 3, dadurch gekennzeichnet, daß der dritte Spiegel (96) in der Beleuchtungseinheit (4) im wesentlichen senkrecht zur Beobachtungsebene verschiebbar ist.

5. Ophthalmoskop nach einem der Ansprüche 1 bis 3, dadurch gekennzeichnet, daß die Verbindungseinrichtung (6) eine Verschiebung der Beleuchtungseinheit (4) im wesentlichen senkrecht zur Beobachtungsebene realtiv zur Beobachtungseinheit (2) zuläßt.

6. Ophthalmoskop nach Anspruch 5, dadurch gekennzeichnet, daß die Verbindungseinrichtung (6) einen an der Beobachtungseinheit (2) befestigten, im wesentlichen senkrecht zur Beobachtungsebene verlaufenden Arm (112) aufweist, der verschiebbar in einer Führung (114) an der Beleuchtungseinheit (4) sitzt.

7. Ophthalmoskop nach Anspruch 6, gekennzeichnet durch eine Feder (128) und eine Druckscheibe (126), die von der Feder gegen den Arm (112) in der Führung (114) gedrückt wird.

8. Ophthalmoskop nach einem der Ansprüche 1 bis 7, gekennzeichnet durch eine im Gehäuse (14) der Beobachtungseinheit (2) ausgebildete Führungsnut (50), die parallel zur Winkelhalbierenden der Beobachtungsstrahlengänge (44, 46, 66, 68) verläuft, einen Träger (54), an dem die zwei ersten Spiegel (32, 34) befestigt sind, einen am Träger ausgebildeten Vorsprung (52), der in die Führungsnut eingreift, und ein außen an der Beobachtungseinheit (2) angeordnetes Betätigungselement (62), das mit dem Träger verbunden ist.

9. Ophthalmoskop nach einem der Ansprüche 1 bis 8, gekennzeichnet durch eine verstellbare Blende (92) und eine Objektivlinse (106) in der Beleuchtungseinheit (4), wobei die Blende in der dingseitigen Brennebene der Objektivlinse angeordnet ist.

**Claims**

1. A binocular ophthalmoscope for indirect observation, comprising an observation unit (2) and an illumination unit (4) connected to said observation unit by means of a connecting means (6), said observation unit (2) deflecting by means of a first (32, 34) and a second (36, 38) mirror, respectively, two observation beam paths (44, 46; 66, 68), entering closely together said observation unit and defining an observation plane, in such a way that their distance is increased to the interpupillary distance of the observer, said two first mirrors (32, 34) being arranged close to each other between said two second mirrors (36, 38) so that they form an optical wedge, and said illumination unit (4) comprising a light source (82) and a third mirror (96) on the emergent side directing light from said light source along an illumination beam path (90, 132) to the eye to be examined characterized in that said two first mirrors (32, 34) are shiftable together within said observation unit (2) to and from the eye to be examined in the observation plane, and in that said illumination beam path (90, 132) is shiftable in a direction perpendicular to said observation plane to and form said observation plane.

2. An ophthalmoscope as claimed in claim 1, characterized in that said third mirror (96) is pivotable about an axis in parallel with said observation plane.

3. An ophthalmoscope as claimed in claim 1, characterized in that between said light source (82) and said third mirror (96) a fourth mirror (98) is arranged in said illumination unit (4) that is pivotable about an axis (100) in parallel with said observation plane.

4. An ophthalmoscope as claimed in one of the claims 1 to 3, characterized in that said third mirror (96) is shiftable in said illumination unit (4) substantially perpendicular to said observation plane.

5. An ophthalmoscope as claimed in one of the claims 1 to 3, characterized in that said connecting means (6) permits a shifting of said illumination unit (4) in a direction substantially perpendicular to said observation plane relative to said observation unit (2).

6. An ophthalmoscope as claimed in claim 5, characterized in that said connecting means (6) comprises an arm (112) mounted on said observation unit (2) and extenting substantially perpendicular to said observation plane, said arm being received slidably in a guide means (114) at said illumination unit (4).

7. An ophthalmoscope as claimed in claim 6, characterized by a spring (128) and a pressure disk (126) which is pressed against said arm (112) in said guide means (114) by said spring.

8. An ophthalmoscope as claimed in one of the claims 1 to 7, characterized by a guiding groove (50) formed in the casing (14) of said observation unit (2) and extending in parallel with the angle bisector of said observation beam paths (44, 46, 66, 68), by a support (54) on which are mounted said first two mirrors (32, 24), by a projection (52) formed at said support and engaging with said guiding groove, and by an operating element (62) arranged on the outer surface of said observation unit (2) and being connected to said support.

9. An ophthalmoscope as claimed in one of the claims 1 to 8, characterized by an adjustable diaphragm (92) and an objective lens (106) in said illumination unit (4), said diaphragm being arranged in the object-side focal plane of said objective lens.

## Revendications

1. Ophthalmoscope binoculaire à observation indirecte, avec une unité d'observation (2) et une unité d'éclairage (4) rendue solidaire de la précédente par un dispositif de liaison (6), dans lequel l'unité d'observation (2), dévie, à l'aide pour chacun d'eux d'un premier (32 – 34) et d'un second miroir (36 – 38) deux faisceaux de rayons lumineux (44 – 46 – 66 – 68) correspondants qui y pénètrent étroitement rapprochés l'un de l'autre en définissant un plan d'observation de manière telle que l'écartement de ces faisceaux se trouve agrandi pour atteindre celui des pupilles des yeux de l'observateur, les deux premiers miroirs (32 – 34) étant disposés étroitement l'un contre l'autre entre les deux seconds (36 – 38) sous la forme d'un coin, tandis que l'unité déclairage (4) comprend une source lumineuse (82) et un troisième miroir (96) situé sur sa sortie, qui renvoie la lumière de la source suivant un faisceau lumineux d'éclairage (90 – 132) vers l'oeil à observer, caractérisé en ce que les deux premiers miroirs (32, 34) sont déplaçables ensemble, à l'intérieur de l'unité d'observation (2) dans le plan d'observation précité dans le sens de rapprochement et de l'éloignement par rapport à l'oeil à observer et en ce que le faisceau lumineux d'éclairgae (90, 132) peut être déplacé dans une direction parallèle audit plan.

2. Ophthalmoscope suivant la revendication 1, caractérisé en ce que le troisième miroir (96) peut basculer autour d'un axe parallèle au plan d'observation.

3. Ophthalmoscope suivant la revendication 1, caractérisé en ce que dans l'unité d'éclairage (4), entre la source lumineuse (82) et le troisième miroir (96) est disposé un quatrième miroir (98) susceptible de basculer autour d'un axe (100) parallèle au plan d'observation.

4. Ophthalmoscope suivant l'une des revendications 1 à 3, caractérisé en ce que le troisième miroir (96) prévu dans l'unité d'éclairage (4) peut être déplacé dans un sens substantiellement perpendiculaire au plan d'observation.

5. Ophthalmoscope suivant l'une des revendications 1 à 3, caractérisé en ce que le dispositif de liaison (6) permet un déplacement de l'unité d'éclairage (4) par rapport à l'unité d'observation (2) dans un sens substantiellement perpendiculaire au plan d'observation précité.

6. Ophthalmoscope suivant la revendication 5, caractérisé en ce que le dispositif de liaison (6) comporte un bras (12) fixé à l'unité d'observation (2) et substantiellement perpendiculaire au plan d'observation, ce bras étant logé à coulissement dans un guidage (114) de l'unité d'éclairage (4).

7. Ophthalmoscope suivant la revendication 6, caractérisé par un ressort (128) et une rondelle de pression (126) qui est appliquée par le ressort contre le bras (112) à l'intérieur du guidage (114).

8. Opthalmoscope suivant l'une des revendications 1 à 7, caractérisé par une rainure de guidage (50) réalisée dans le boîtier (14) de l'unité d'observation (2) et qui s'étend parallèlement à la bissectrice de l'angle des faisceaux lumineux d'observation (44, 46, 66, 68), par un support (54) auquel sont fixés les deux premiers miroirs (32, 34), par une saillie (52) qui s'engage dans la rainure de guidage, et par un élément d'actionnement (62) disposé extérieurement à l'unité d'observation (2) et qui est fixé au support.

9. Ophthalmoscope suivant l'une des revendications 1 à 8, caractérisé par un diaphragme réglable (92) et par une lentille-objectif (106), prévus à l'intérieur de l'unité d'éclairage (4), le diaphragme étant disposé au foyer-objet de cet objectif.

Fig. 1

0 041 107

_Fig.3_

_Fig.4_

0 041 107